# EUROPEAN PATENT SPECIFICATION

(11) **EP 3 869 446 B1**
(45) Date of publication and mention of the grant of the patent: **10.04.2024**
(21) Application number: 18937195.8
(22) Date of filing: 21.12.2018
(51) Int. Cl.: G06T 5/00, G06T 11/00, A61B 6/03, A61B 6/00

(54) **APPARATUS AND METHOD FOR DEEP LEARNING-BASED CT IMAGE NOISE REDUCTION**
VORRICHTUNG UND VERFAHREN FÜR AUF TIEFENLERNEN BASIERENDER CT-BILDRAUSCHUNTERDRÜCKUNG
APPAREIL ET PROCÉDÉ DE RÉDUCTION DE BRUIT D'IMAGE CT À BASE D'APPRENTISSAGE APPROFONDI

(30) Priority: 17.10.2018 KR 20180123786
(43) Date of publication of application: 25.08.2021
(73) Proprietor: Claripi Inc., Seoul 03088 (KR); Seoul National University R & DB Foundation, Seoul 08826 (KR)
(72) Inventor: PARK, Hyun Sook, Seoul 02861 (KR); KIM, Jong Hyo, Seoul 02861 (KR)
(74) Representative: Schmitt-Nilson Schraud Waibel Wohlfrom Patentanwälte Partnerschaft mbB
(86) International application number: PCT/KR2018/016451
(87) International publication number: WO 2020/080604

(56) References cited:
- KR-A- 20140 042 531
- KR-A- 20180 004 824
- KR-A- 20180 040 287
- KR-B1- 101 697 501
- US-A1- 2015 201 895
- OZAN OKTAY ET AL: "Anatomically Constrained Neural Networks (ACNN): Application to Cardiac Image Enhancement and Segmentation", ARXIV.ORG, CORNELL UNIVERSITY LIBRARY, 201 OLIN LIBRARY CORNELL UNIVERSITY ITHACA, NY 14853, 22 May 2017 (2017-05-22), XP081404620, DOI: 10.1109/TMI.2017.2743464
- GONDARA LOVEDEEP: "Medical Image Denoising Using Convolutional Denoising Autoencoders", 2016 IEEE 16TH INTERNATIONAL CONFERENCE ON DATA MINING WORKSHOPS (ICDMW), IEEE, 12 December 2016 (2016-12-12), pages 241-246, XP033055819, DOI: 10.1109/ICDMW.2016.0041 [retrieved on 2017-01-30]

## Description

### [Technical Field]

The present disclosure relates to a deep learning based CT image noise reducing apparatus and method.

### [Background Art]

When computed tomographic scanning (CT) is used, a subject enters a large circular machine with an X-ray generator to be scanned so that a cross-sectional image intersecting a human body may be obtained. It has an advantage in that structures are less overlaid than in simple X-ray imaging so that structures and lesions may be more clearly observed. Therefore, the CT is widely utilized for a complete check-up for most organs and diseases.

A quality (a resolution and a precision) of a CT image is a very important factor in precise diagnosis for lesions and efforts to improve the quality of the CT image are continuing together with the development of the CT system. For example, a multichannel detector technique and a high speed and high resolution image reconstruction technique may be regarded as such efforts. However, the efforts of the related art for improving the quality of the CT image may cause a high dose of radiation exposure and damages thereof are concerned. Specifically, when the recent social perception of radiation exposure is considered, the efforts for acquiring a high quality diagnostic image need to be accompanied by efforts for minimizing the radiation dose.

As an example of such efforts, CT manufacturers are releasing high quality CT systems with reduced radiation exposure. However, the high quality CT systems with reduced radiation exposure of the related art have problems in that the price is higher than that of the existing products and troubles to dispose of the existing products are caused. As another example of the efforts, the CT manufacturers may acquire high quality CT images with reduced radiation exposure by upgrading hardware and software of the existing products of each manufacturer. However, this also cannot be an actual solution considering a considerable upgrade cost so that a solution therefor is required. That is, it is required to develop a more effective technique to acquire a high quality diagnostic image (CT image) while minimizing a radiation dose.

In the meantime, the deep learning technology is a new type of computing technique which achieves a specific purpose using a general-purpose program code which can be trained according to a given learning data set, instead of coding a series of detailed programs and its excellent performance is recognized in various image processing fields.

However, in order to show the desirable performance of the deep learning model, it is not only required to ensure enough training data sets, but also needs to be accompanied by a method of partitioning data to allow the deep learning model to be trained for data in a previously designated range and to be operated for data in the previously designated range even in an actual usage stage. In order to apply the deep learning to medical images in which safety is particularly important, it can be said that the development of the effective deep learning training technology which satisfies such prerequisites is even more important.

A background art of the present disclosure is disclosed in Korean Unexamined Patent Application Publication No. 10-2014-0130784.

OZAN OKTAY ET AL, "Anatomically Constrained Neural Networks (ACNN): Application to Cardiac Image Enhancement and Segmentation", ARXIV.ORG, CORNELL UNIVERSITY LIBRARY, 201 OLIN LIBRARY CORNELL UNIVERSITY ITHACA, NY 14853, (20170522), doi:10.1109/TMI.2017.2743464, discloses a generic training strategy that incorporates anatomical prior knowledge into constrained neural networks (CNNs) through a new regularisation model, which is trained end-to-end, which encourages models to follow the global anatomical properties of the underlying anatomy (e.g. shape, label structure) via learnt non-linear representations of the shape.

### [Disclosure]

### [Technical Problem]

The present disclosure has been made an effort to solve the problems of the related art and an object thereof is to provide a deep learning based CT image noise reducing apparatus and method which obtain a high quality diagnostic image (CT image) while minimizing a radiation dose.

The present disclosure has been made an effort to solve the problems of the related art and an object of the present disclosure is to provide a training (learning) method of a deep learning model by means of a deep learning based CT image noise reducing apparatus to more effectively apply the deep learning to medical images in which the safety is specifically important.

The present disclosure has been made an effort to solve the problems of the related art and an object of the present disclosure is to provide a deep learning based CT image noise reducing apparatus and method and a training method of a deep learning model therethrough which ensure the performance and the safety of the deep learning while utilizing the advantages of the deep learning.

However, technical objects to be achieved by various embodiments of the present disclosure are not limited to the technical objects as described above and other technical objects may be present.

### [Technical Solution]

The present invention is defined by independent claims and various aspects of the invention are disclosed in dependent claims, the description, and the drawings.

As a technical means to achieve the above-described technical object, according to an aspect of the present disclosure, a deep learning based CT image noise reducing method includes: extracting test information from an input CT image; selecting at least one deep learning model corresponding to the test information, among a plurality of previously trained deep learning models; and outputting a CT image with a reduced noise with respect to the input CT image with the input CT image as an input of at least one selected deep learning model.

Further, according to an aspect of the present disclosure, the deep learning based CT image noise reducing method may further include: before the extracting, generating a second CT data set for training to which various levels of noises are added by applying a CT image noise simulator to a first CT data set for training; extracting test information from the second CT data set for training and grouping the second CT data set for training into a plurality of groups according to a predetermined rule; and generating and training a plurality of deep learning models to be trained so as to correspond to each grouped second CT data set for training for every group, and in the selecting, the plurality of previously trained deep learning models may be a plurality of deep learning models to be trained which is trained by the training.

Further, in the generating, the CT image noise simulator may perform generating a composite Sinogram with an original image of the first CT data set for training as an input; generating a noise component composite Sinogram by applying a noise model with a predetermined level of noise to the composite Sinogram; generating a noise component CT image based on the generated noise component composite Sinogram; and generating a composite high noise CT image by adding the generated noise component CT image and the original CT image.

Further, the second CT data set for training may be formed of a pair of a composite high noise CT image and a composite noise component CT image obtained based on an original image of the first CT data set for training.

Further, in the training, in order to allow the plurality of deep learning models to be trained to have a function of extracting the noise component CT image from the input CT image input thereto, a composite high noise CT image for every CT image may be transmitted as an input of the deep learning model to be trained, in the grouped second CT data set for training for every group and the deep learning model to be trained may be repeatedly trained so as to minimize a difference between the composite noise component CT image and the output of the deep learning model to be trained.

Further, in the outputting, at least one selected deep learning model extracts a noise component CT image from the input CT image with the input CT image as an input of the at least one selected deep learning model and a predetermined value may be multiplied with the extracted noise component CT image to be subtracted from the input CT image to output the CT image with a reduced noise.

Further, according to another aspect of the present disclosure, a deep learning based CT image noise reducing apparatus includes: an extracting unit which extracts test information from an input CT image; a selecting unit which selects at least one deep learning model corresponding to the test information, among a plurality of previously trained deep learning models; and an ouput unit which outputs an input CT image with a reduced noise with respect to the input CT image with the input CT image as an input of the at least one selected deep learning model.

Further, according to an aspect of the present disclosure, the deep learning based CT image noise reducing apparatus may further include: a training unit which generates and trains a plurality of deep learning models to be trained, the training unit may generate a second CT data set for training to which a noise is added by applying a CT image noise simulator to a first CT data set for training, extract test information from the second CT data set for training and group the second CT data set for training into a plurality of groups according to a predetermined rule, and generate and train a plurality of deep learning models to be trained so as to correspond to each grouped second CT data set for training for every group, and the plurality of previously trained deep learning models may be the plurality of deep learning models to be trained which is trained by the training unit.

Further, the CT image noise simulator may generate a composite Sinogram with an original image of the first CT data set for training as an input, generate a noise component composite Sinogram by applying a noise model with a predetermined level of noise to the composite Sinogram, generate a noise component CT image based on the generated noise component composite Sinogram, and generate a composite high noise CT image by adding the generated noise component CT image and the original CT image.

Further, the second CT data set for training may be formed of a pair of a composite high noise CT image and a composite noise component CT image obtained based on an original image of the first CT data set for training.

In order to allow the plurality of deep learning models to be trained to have a function of extracting the noise component CT image from the input CT image input thereto, the training unit may transmit a composite high noise CT image for every CT image as an input of the deep learning model to be trained, in the grouped second CT data set for training for every group and repeatedly train the deep learning model to be trained so as to minimize a difference between the composite noise component CT image and the output of the deep learning model to be trained.

Further, the output unit allows the at least one selected deep learning model to extract a noise component CT image from the input CT image with the input CT image as an input of the at least one selected deep learning model and multiply a predetermined value with the extracted noise component CT image to be subtracted from the input CT image to output the CT image with a reduced noise.

The above-described solving means are merely illustrative but should not be construed as limiting the present disclosure. In addition to the above-described embodiments, additional embodiments may be further provided in the drawings and the detailed description of the present disclosure.

### [Advantageous Effects]

According to the above-described solving means of the present disclosure, the noise of the CT image is reduced based on deep learning to acquire a high quality diagnostic image (CT image) while minimizing a radiation dose.

According to the above-described solving means of the present disclosure, the plurality of deep learning models (a plurality of deep learning models to be trained) is trained according to the test information of the CT image to effectively apply the deep learning to a medical image in which the safety is specifically important.

According to the above-described solving means of the present disclosure, a deep learning based CT image noise reducing apparatus, method, and a deep learning model training (learning) method therethrough which ensure the performance of the deep learning and secure the safety while utilizing the advantages of the deep learning may be provided.

However, the effect which can be achieved by the present disclosure is not limited to the above-described effects, there may be other effects.

### [Description of Drawings]

FIG. 1 is a view illustrating a schematic configuration of a deep learning based CT image noise reducing apparatus according to an exemplary embodiment of the present disclosure.
FIG. 2 is a view illustrating a schematic operation flow for a deep learning based CT image noise reducing method by a deep learning based CT image noise reducing apparatus according to an exemplary embodiment of the present disclosure.
FIG. 3 is a view illustrating an example of a schematic operation flow for a deep learning model training method for reducing a CT image noise based on deep learning by a deep learning based CT image noise reducing apparatus according to an exemplary embodiment of the present disclosure.

### [Best Mode]

Hereinafter, the present disclosure will be described more fully hereinafter with reference to the accompanying drawings, in which exemplary embodiments of the present disclosure are shown. However, the present disclosure can be realized in various different forms, and is not limited to the embodiments described herein. Accordingly, in order to clearly explain the present disclosure in the drawings, portions not related to the description are omitted. Like reference numerals designate like elements throughout the specification.

Throughout this specification and the claims that follow, when it is described that an element is "coupled" to another element, the element may be "directly coupled" to the other element or "electrically coupled" or "indirectly coupled" to the other element through a third element.

Through the specification of the present disclosure, when one member is located "on", "above", "on an upper portion", "below", "under", and "on a lower portion" of the other member, the member may be adjacent to the other member or a third member may be disposed between the above two members.

In the specification of the present disclosure, unless explicitly described to the contrary, the word "comprise" and variations such as "comprises" or "comprising", will be understood to imply the inclusion of stated elements but not the exclusion of any other elements.

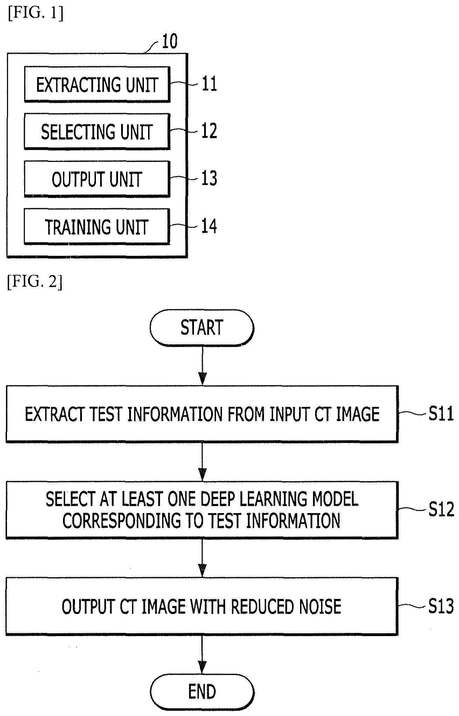

FIG. 1 is a view illustrating a schematic configuration of a deep learning based CT image noise reducing apparatus according to an exemplary embodiment of the present disclosure. Hereinafter, the deep learning based CT image noise reducing apparatus 10 according to an exemplary embodiment of the present disclosure is referred to as this apparatus 10 for the convenience of description.

Referring to FIG. 1, this apparatus 10 may include an extracting unit 11, a selecting unit 12, an output unit 13, and a training unit 14.

The extracting unit 11 may extract test information from an input CT image. Specifically, the extracting unit 11 may extract the test information from header information included in the input CT image. The test information may include test portion information and scan attribute information of the CT image.

Here, the test portion information may refer to, for example, information about an organ portion. That is, the test portion information refers to information about the organs of the human body of major interest, and may refer to, for example, information about organs such as head, chest, and abdomen. Further, the scan attribute information refers to information about CT scanning variables which influence a noise characteristic of the CT image and for example, may refer to information such as a reconstruction kernel, a tube voltage, a tube current, and a slice thickness.

The input CT image may be an image acquired by computed tomography (CT), magnetic resonance imaging (MRI), positron emission tomography (PET), or the like, but is not limited thereto and may be an image acquired by various imaging devices.

The selecting unit 12 may select at least one deep learning model corresponding to test information extracted by the extracting unit 11, among a plurality of previously-trained deep learning models.

The selecting unit 12 applies a predetermined rule to the test information extracted by the extracting unit 11 to select at least one (one or more) deep learning model corresponding to the test information extracted by the extracting unit 11, among the plurality of previously trained deep learning models.

According to this, this apparatus 10 may include a plurality of deep learning models. Here, each of the plurality of deep learning models is a deep learning model which has been trained in advance by the training unit 14 which will be described below and specifically, refers to a deep learning model which has been trained in advance so as to output a CT image with a reduced noise (a noise reduced CT image) with respect to a CT image (an input CT image) input to each deep learning model.

According to another exemplary embodiment, each of the plurality of deep learning models included in this apparatus 10 may refer to a deep learning model which has been trained in advance to extract a noise component CT image with respect to a CT image (in other words, an input CT image input thereto) input to each deep learning model.

The plurality of deep learning models considered in this apparatus 10 may be generated by the training unit 14. The training unit 14 may generate a plurality of deep learning models according to the test information. Specifically, the training unit 14 may generate a plurality of deep learning models according to a combination of the test portion information and the scan attribute information of the CT image included in the test information.

In other words, this apparatus 10 may include a plurality of deep learning models generated according to the test information (according to the combination of the test portion information and the scan attribute information). The plurality of deep learning models generated according to the combination of the test portion information and the scan attribute information may be trained by the training unit 14 using each CT data set for training (that is, a CT data set for deep learning training) grouped according to the combination of the test portion information and the scan attribute information.

When the deep learning model is described in the present disclosure, a deep learning model before being trained by the training unit 14 may be referred to as a deep learning model to be trained. According to this, after completing the training for the deep learning model to be trained, the deep learning model means a previously trained deep learning model. In other words, the plurality of previously trained deep learning models considered in the selecting unit 12 may refer to a plurality of deep learning models to be trained which has been trained by the training unit 14. The training of the deep learning model to be trained will be described in more detail below.

The selecting unit 12 may select at least one deep learning model corresponding to the test information extracted by the extracting unit 11 (corresponding to the combination of the test portion information and the scan attribute information extracted by the extracting unit), among the plurality of deep learning models which is generated according to the test information (according to the combination of the test portion information and the scan attribute information) and is trained in advance.

The selecting unit 12 may select at least one deep learning model which is suitable to apply an input CT image, among a plurality of previously trained deep learning models, based on the test information extracted by the extracting unit 11.

For example, it is assumed that the input CT image is an abdomen test CT image which is a CT image of a first test portion (for example, an abdomen) which is reconstructed with a first scan attribute (for example, a scan attribute of a low dose and a thick slice). In this case, the selecting unit 12 may select a single deep learning model (for example, a first deep learning model) trained with training data (a CT data set for training) with an abdomen as a test portion and a scan attribute of a low dose and a thick slice, as a deep learning model corresponding to the first scan attribute and the first test portion, among the plurality of previously trained deep learning models.

As another example, it is assumed that the input CT image is an abdomen test CT image which is a CT image of a first test portion (for example, an abdomen) which is reconstructed with a second scan attribute (for example, a scan attribute of an ultra-low dose and a thin slice). In this case, the selecting unit 12 may select a single deep learning model (for example, a second deep learning model) trained with training data (a CT data set for training) with an abdomen as a test portion and a scan attribute of an ultra-low dose and a thin slice, as a deep learning model corresponding to the second scan attribute and the first test portion, among the plurality of previously trained deep learning models.

As still another example, it is assumed that the input CT image is a chest test CT image which is a CT image of a second test portion (for example, a chest) reconstructed by applying a third scan attribute (for example, a reconstruction kernel with a high sharpness as a first sharpness is applied to a lung area) to a first area and applying a fourth scan attribute (for example, a reconstruction kernel with a low sharpness as a second sharpness is applied to a soft tissue area) to a second area. In this case, the selecting unit 12 may select two deep learning models including a third deep learning model corresponding to the third scan attribute and the second test portion and a fourth deep learning model corresponding to the fourth scan attribute and the second test portion, among the plurality of previously trained deep learning models. Specifically, the selecting unit 12 may select two deep learning models including the third deep learning model which is trained with training data (a CT data set for training) in which a test portion is a chest and a reconstruction kernel has a high sharpness and the fourth deep learning model which is trained with training data (a CT data set for training) in which a test portion is a chest and a reconstruction kernel has a low sharpness, among the plurality of previously trained deep learning models.

As described above, the selecting unit 12 may select one or more deep learning models (at least one deep learning model) among the plurality of previously trained deep learning models in consideration of the combination of the test portion information and the scan attribute information included in the extracted test information, based on the test information extracted by the extracting unit 11.

The output unit 13 may output an input CT image obtained by reducing a noise of the input CT image with the input CT image as an input of at least one deep learning model selected by the selecting unit 12. That is, the output unit 13 may input an input CT image to at least one deep learning model selected by the selecting unit 12 and then output an input CT image obtained by reducing a noise of the input CT image through an output of at least one selected deep learning model.

The output unit 13 allows at least one selected deep learning model to extract a noise component CT image from the input CT image, with the input CT image as an input of at least one deep learning model selected by the selecting unit 12 and multiplies a predetermined value (a predetermined ratio) with the extracted noise component CT image to be subtracted from the input CT image to output a CT image with a reduced noise with respect to the input CT image by means of at least one selected deep learning model.

As an example, it is assumed that a deep learning model selected by the selecting unit 12 is a single (one) deep learning model. In this case, the output unit 13 inputs the input CT image to the single deep learning model selected by the selecting unit 12 to allow the single deep learning model to extract the noise component CT image of the input CT image and multiplies the predetermined ratio with the extracted noise component CT image to be subtracted from the input CT image, thereby outputting a CT image with a reduced noise with respect to the input CT image.

As another example, it is assumed that the deep learning model selected by the selecting unit 12 is a plurality of deep learning models. In this case, the output unit 13 inputs the input CT image to the plurality of deep learning models selected by the selecting unit 12 to allow the plurality of deep learning models to extract the noise component CT image from the input CT image input to the plurality of deep learning models and mixes a plurality of noise component CT images extracted by the plurality of deep learning models according to a predetermined rule to be subtracted from the input CT image, thereby outputting a CT image with a reduced noise with respect to the input CT image.

The training unit 14 may generate and train the plurality of deep learning models to be trained before extracting the test information from the input CT image by the extracting unit 11.

In this case, the training unit 14 may generate a second CT data set for training with a noise by applying a CT image noise simulator to the first CT data set for training, extract test information from the second CT data set for training, group the second CT data sets for training into a plurality of groups according to a predetermined rule, and generate and train a plurality of deep learning models to be trained so as to correspond to each of the grouped second CT data sets for training for every group. The training unit 14 may individually train the plurality of generated deep learning models to be trained.

At this time, the plurality of deep learning models to be trained which is trained by the training unit 14 may refer to a plurality of previously trained deep learning models which is considered in the selecting unit 12.

Further, the CT image noise simulator, which is considered when the second CT data set for training is generated in the training unit 14, may generate a composite Sinogram with an original image of the first CT data set for training as an input, generate a noise component composite Sinogram by applying a noise model having a predetermined level (a desired level) of noise to the composite Sinogram, generate a nose component CT image based on the generated noise component composite Sinogram, and generate a composite high noise CT image by adding the generated noise component CT image and the original CT image.

Further, the second CT data set for training which is considered in the training unit 14 may be formed of a pair of a composite high noise CT image and a composite noise component CT image obtained based on an original image of the first CT data set for training (in other words, derived from the original image).

According to this, the training unit 14 may train the deep learning to be trained using the pair of the composite high noise CT image and the composite noise component CT image obtained by applying the CT image noise simulator to the original image of the first CT data set for training.

Further, in order to allow the plurality of deep learning models to be trained to have a function of extracting the noise component CT image from the input CT image input thereto, the training unit 14 transmits a composite high noise CT image for every CT image as an input of the deep learning model to be trained, in the grouped second CT data set for training for every group and repeatedly trains the deep learning model to be trained so as to minimize a difference between the composite noise component CT image and the output of the deep learning model to be trained.

Hereinafter, the training unit 14 will be described in more detail.

The training unit 14 may train the deep learning model to be trained using a CT data set for training (in other words, a CT data set for deep learning training) generated by means of the CT image noise simulator, before extracting the test information from the input CT image by the extracting unit 11. Here, the deep learning model to be trained may refer to at least one deep learning model.

The training unit 14 applies an original CT image of the first CT data set for training as an input of the CT image noise simulator to generate a second CT data set for training with various levels of noises, before training the deep learning model to be trained. Next, the training unit 14 may train the deep learning model to be trained using the generated second CT data set for training.

In this case, the training unit 14 may train the deep learning models to be trained corresponding to the deep learning models so as to allow the deep learning models included in this apparatus 10 to output a low noise CT image with a reduced noise using the generated second CT data set for training with the high noise CT image as an input. In other words, when the deep learning models included in this apparatus 10 receive the high noise CT image by training the deep learning model to be trained by the training unit 14, the deep learning model may be trained to output a low noise input CT image.

After generating the second CT data set for training to which various levels of noises are added, the training unit 14 may extract the test information (test information including test portion information and scan attribute information) from the second CT data set for training and group the second CT data set for training into a plurality of groups according to a predetermined rule. By doing this, a plurality of second CT data set groups for training which is grouped according to the predetermined rule may include a second CT data set for training for every test portion and a second CT data set for training for every scan attribute information.

Specifically, when the CT data set for training is generated, the training unit 14 may generate a CT data set for training for every noise level. In other words, when the training unit 14 generates the second CTI data set for training to which various levels of noises are added, the training unit 14 may generate different second CT data sets for training (second CT data sets for deep learning training) depending on a plurality of predetermined noise levels.

Thereafter, the training unit 14 may extract the test information including the test portion information and the scan attribute information from the second CT data set for training generated for every noise level. Thereafter, the training unit 14 may group the second CT data sets for training into a plurality of groups according to a predetermined rule based on the extracted test information. Here, the predetermined rule may refer to a rule for dividing the CT data (CT images) included in the second CT data sets for training generated for every noise level, for every predetermined test portion and/or every predetermined scan attribute.

By doing this, the training unit 14 may divide the second CT data sets for training generated for every noise level, for every predetermined test portion and/or every predetermined scan attribute into a plurality of groups. That is, a plurality of second CT data set groups for training which is grouped by the grouping may include a second CT data set for training for every test portion and a second CT data set for training for every scan attribute.

Thereafter, the training unit 14 may generate a plurality of deep learning models to be trained so as to correspond to each of the second CT data sets for training for every group which is grouped into a plurality of groups. That is, by doing this, the plurality of deep learning models to be trained generated by the training unit 14 may include a plurality of deep learning models to be trained generated for every test portion and a plurality of deep learning models to be trained generated for every scan attribute.

Thereafter, the training unit 14 may train the plurality of generated deep learning models for training with the second CT data sets for training generated so as to correspond thereto.

That is, the training unit 14 may train the deep learning models to be trained generated for every test portion using the CT data set for training generated for every test portion. Further, the training unit 14 may train each of the deep learning models to be trained generated for every scan attribute using the CT data set for training generated for every scan attribute.

In other words, the training unit 14 may train the deep learning models to be trained generated for every test portion using corresponding CT data sets for training generated for every test portion. Further, the training unit 14 may train each of the deep learning models to be trained generated for every scan attribute using corresponding CT data sets for training generated for every scan attribute.

By doing this, the training unit 14 may train the plurality of deep learning models to be trained (a plurality of deep learning models) using the plurality of CT data sets for training generated for every test portion and every scan attribute (that is, second CT data sets for training for every group which are grouped into a plurality of groups) so as to allow each of the plurality of deep learning models to be trained (the plurality of deep learning models) included in this apparatus 100 generated for every test portion and every scan attribute to output a low noise CT image with a reduced noise with a high noise CT image as an input.

After generating the plurality of deep learning model to be trained so as to correspond to each of second CT data sets for training for every group which is grouped into a plurality of groups, the training unit 14 may train the plurality of deep learning models to be trained using the CT data set for training generated so as to correspond thereto.

When each of the plurality of deep learning models included in this apparatus 10 receives a high noise CT image as an input CT image, the plurality of deep learning models may be trained (learned) to output a low noise input CT image with a reduced nose with respect to the high noise CT image, by means of the training by the training unit 14.

In other words, the training unit 14 receives an original CT image using the CT image noise simulator to generate different CT data sets for training (second CT data sets for training for every noise level) according to a plurality of noise levels.

Thereafter, the training unit 14 may group the CT data sets for training generated, for example, for every noise level into a plurality of groups for every scan attribute. That is, the training unit 14 may generate a plurality of CT data set groups for training including the CT data set for training for every scan attribute based on the CT data set for training generated for every noise level.

Next, the training unit 14 may generate a plurality of deep learning models to be trained for every scan attribute as a deep learning model to be trained included in this apparatus 10 so as to correspond to each of the second CT data sets for training generated, for example, for every scan attribute.

Next, the training unit 14 may train each of the deep learning models to be trained generated for every scan attribute using the CT data set for training (that is, a CT data set for training for every scan attribute) generated so as to correspond to the corresponding scan attribute. In this case, when the input CT image is given as an input, the training unit 14 may train each of the plurality of deep learning models to be trained generated for every scan attribute so as to allow the deep learning model (a deep learning model to be trained) corresponding to a scan attribute of the input CT image to output a low noise CT image with the input CT image as an input.

By doing this, when an input CT image is given in this apparatus 10, the selecting unit 12 may select a deep learning model corresponding to the scan attribute of the input CT image so as to operate a corresponding deep learning model according to the scan attribute of the input CT image, among the plurality of previously trained deep learning models and then the output unit 13 may output an input CT image with a reduced noise using the selected deep learning model.

Further, the training unit 14 may receive an original CT image using the CT image noise simulator to generate different CT data sets for training (second CT data sets for training for every noise level) according to a plurality of noise levels.

Thereafter, the training unit 14 may group the CT data sets for training generated, for example, for every noise level into a plurality of groups for every test portion. That is, the training unit 14 may generate a plurality of CT data set groups for training including the CT data set for training for every test portion based on the CT data set for training generated for every noise level.

Next, the training unit 14 may generate a plurality of deep learning models to be trained for every test portion as a deep learning model to be trained included in this apparatus 10 so as to correspond to each of the second CT data set for training generated, for example, for every test portion.

Next, the training unit 14 may train each of the deep learning models to be trained generated for every test portion using the CT data set for training (that is, a CT data set for training for every test portion) generated so as to correspond to the corresponding test portion. In this case, when the input CT image is given as an input, the training unit 14 may train each of the plurality of deep learning models to be trained generated for every test portion so as to allow the deep learning model (a deep learning model to be trained) corresponding to a test portion of the input CT image to output a low noise CT image with the input CT image as an input.

By doing this, when an input CT image is given in this apparatus 10, the selecting unit 12 may select a deep learning model corresponding to the test portion of the input CT image so as to operate a corresponding deep learning model according to the test portion of the input CT image, among the plurality of previously trained deep learning models and then the output unit 13 may output an input CT image with a reduced noise using the selected deep learning model.

That is, the CT data set for training for every test portion and/or every scan attribute is generated and the deep learning model to be trained for every test portion and/or every scan attribute may be generated and trained, by the training unit 14.

Further, the CT data set for training generated by the training unit 14 may include at least one pair of an original CT image, a composite high noise CT image, and a composite noise component CT image. In other words, the training unit 14 may generate a CT data set for training (a second CT data set for training) including at least one pair of the original CT image, the composite high noise CT image, and the composite noise component CT image, with the original CT image as an input of the CT image noise simulator.

The second CT data set for training which is considered in the training unit 14 may be formed of a pair of a composite high noise CT image and a composite noise component CT image derived from an original image of the first CT data set for training.

The training unit 14 may transmit the composite high noise CT image as an input of the deep learning model to be trained and repeatedly train the deep learning model to be trained so as to minimize the difference between the composite noise component CT image and the output of the deep learning model to be trained, for every CT image.

That is, in order to allow the plurality of deep learning models to be trained to have a function of extracting the noise component CT image from the input CT image input thereto, the training unit 14 may transmit a composite high noise CT image for every CT image as an input of the deep learning model to be trained, in the grouped second CT data set for training for every group and repeatedly train the plurality of deep learning models to be trained so as to minimize a difference between the composite noise component CT image and the output of the deep learning model to be trained.

In other words, the training unit 14 may transmit the composite high noise CT image to the input of the deep learning model to be trained, for every CT image, in the second CT data set for training divided into groups and repeatedly train the deep learning model to be trained so as to minimize the difference between the composite noise component CT image and the output of the deep learning model to be trained so that the deep learning model to be trained may have a function of extracting a noise component from the CT image.

Further, the CT image noise simulator which is used for training in the training unit 14 may generate a composite Sinogram with an original CT image of the first CT data set for training as an input. Further, the CT image noise simulator applies a noise model having a predetermined level (a desired level) of noise to the generated composite Sinogram to generate a noise component composite Sinogram. Further, the CT image noise simulator may generate a noise component CT image based on the generated noise component composite Sinogram. Further, the CT image noise simulator may generate a composite high noise CT image by adding the generated noise component CT image and the original CT image.

As a specific example, the CT image noise simulator may generate a composite Sinogram with an original CT image of the first CT data set for training as an input. Thereafter, the CT image noise simulator may generate a noise component composite Sinogram by applying a CT image physical principle (that is, a physical principle of a CT image) as the generated composite Sinogram and a noise model with a predetermined level of noise. Thereafter, the CT image noise simulator may generate a noise component CT image based on the generated noise component composite Sinogram. Thereafter, the CT image noise simulator may generate a composite high noise CT image included in the second data set for training by adding the generated noise component CT image and the original CT image.

At this time, when the CT image noise simulator generates a composite Sinogram, the CT image noise simulator may determine an attenuation coefficient per pixel of the original CT image, distance information between an X-ray tube focal point and a detector, and distance information between an X-ray tube focal point and a subject corresponding to an input CT image, based on medical information of the original CT image.

Thereafter, the CT image noise simulator may generate a composite Sinogram based on the determined attenuation coefficient per pixel, distance information between an X-ray tube focal point and a detector, and distance information between an X-ray tube focal point and a subject. Specifically, the CT image noise simulator may generate a composite Sinogram by performing a projection operation at every rotation angle based on the determined attenuation coefficient per pixel, distance information between an X-ray tube focal point and a detector, and distance information between an X-ray tube focal point and a subject.

As described above, this apparatus 10 may train the plurality of deep learning models (that is, a plurality of deep learning models to be trained) by means of the training unit 14 so as to allow the plurality of deep learning models included in this apparatus 10 to output a low noise CT image with a reduced noise with respect to a CT image input to each deep learning model. Based on the plurality of deep learning models which has been trained in advance (a plurality of deep learning models to be trained which is trained by the training unit), this apparatus 10 may select a corresponding deep learning model from the plurality of previously trained deep learning models in consideration of the test information extracted from the input CT image. Thereafter, this apparatus 10 may output an input CT image with a reduced noise (that is, a low noise input CT image) with respect to the input CT image from the selected deep learning model by providing the input CT image to the selected deep learning model as an input.

The present disclosure may provide a deep learning based CT image noise reducing apparatus (this apparatus 10) which outputs a high quality CT image with a reduced noise (an input CT image with a reduced noise or a low noise input CT image) from an input of a low quality (resolution, precision) input CT image (for example, a less exposed input CT image), based on the plurality of previously trained deep learning models. The input CT image with a reduced noise which is output from this apparatus 10 may have a quality which is equal to or higher than a quality of a highly exposed CT image.

Further, this apparatus 10 provides a training (learning) method of the deep learning model (a deep learning model to be trained) to output a high quality CT image with a reduced noise from an input of a low quality input CT image and the input CT image with a reduced noise output by the deep learning model trained as described above (that is, a previously trained deep learning model) may have a quality which is equal to or higher than a quality of the highly exposed CT image.

This apparatus 10 may extract test portion information and scan attribute information as test information from the input CT image using medical information included in the input CT image. Thereafter, this apparatus 10 may select a deep learning model corresponding to the extracted test information (that is, a deep learning model which has been trained in advance to show a predetermined level of performance) from a plurality of previously trained deep learning models and output (acquire) an input CT image with a reduced noise by the selected deep learning model. This apparatus 10 may train each of the plurality of deep learning models to be trained by means of the training unit 14 to allow the plurality of deep learning models to show a predetermined level of performance (a level of minimizing a difference between the composite noise component CT image and an output of the deep learning model to be trained).

Further, this apparatus 10 may receive the original CT image to generate the CT data set for training including the composite high noise CT image using the CT noise simulator and train the deep learning model to be trained included in this apparatus 10 using the generated CT data set for training. The deep learning model to be trained included in this apparatus 10 may be a deep learning model which has been trained in advance by means of the training using the CT data set for training and such a previously trained deep learning model may refer to a deep learning model which has been trained in advance to output a CT image with a reduced noise (a low noise CT image) from the high noise CT image.

Further, this apparatus 10 trains the deep learning model to be trained with the CT data set for training generated using the CT noise simulator so that when the high noise CT image (input CT image) acquired during the actual low-exposure scanning is used as an input of this apparatus 10 (that is, used as an input of at least one deep learning model among the plurality of previously trained deep learning models included in this apparatus), this apparatus may output a low noise CT image with a noise which is more effectively reduced with respect to the high noise CT image.

The plurality of deep learning models generated for every test portion and every scan attribute, included in this apparatus 10, may be trained by the training unit 14 to output a CT with a reduced noise with respect to the original CT image input thereto.

This apparatus 10 may also be represented not only as a deep learning based CT image noise reducing apparatus, but also as a deep learning model (a deep learning model to be trained) training apparatus for reducing a noise of a CT image based on deep learning. The present disclosure may provide not only a deep learning based CT image noise reducing method, but also a deep learning model training method for reducing a CT image noise based on deep learning, by means of this apparatus 10.

Hereinafter, an operation flow of the present disclosure will be described in brief based on the above detailed description.

FIG. 2 is a view illustrating a schematic operation flow for a deep learning based CT image noise reducing method by a deep learning based CT image noise reducing apparatus according to an exemplary embodiment of the present disclosure.

The deep learning based CT image noise reducing method illustrated in FIG. 2 may be performed by the above-described deep learning based CT image noise reducing apparatus (this apparatus, 10). Accordingly, even though description is omitted, the description for the deep learning based CT image noise reducing apparatus (this apparatus, 10) may also be applied to the description for the deep learning based CT image noise reducing method in the same manner.

Referring to FIG. 2, in the deep learning based CT image noise reducing method according to an exemplary embodiment of the present disclosure, the extracting unit 11 may extract test information from an input CT image in step S11.

Next, in step S12, the selecting unit 12 may select at least one deep learning model corresponding to test information extracted in step S11, among a plurality of previously trained deep learning models.

Next, in step S13, the output unit 13 may output a CT image obtained by reducing a noise of the input CT image with the input CT image as an input of at least one deep learning model selected in step S12.

In this case, in step S13, the output unit 13 allows at least one deep learning model selected in step S12 to extract a noise component CT image from the input CT image, with the input CT image as an input of at least one deep learning model selected in step S12 and multiplies a predetermined value (for example, a predetermined ratio) with the extracted noise component CT image to be subtracted from the input CT image to output a CT image with a reduced noise with respect to the input CT image.

In the meantime, the deep learning based CT image noise reducing method according to the exemplary embodiment of the present disclosure may include generating and training a plurality of deep learning models to be trained before step S11. At this time, the plurality of deep learning models to be trained which is trained by the training may refer to a plurality of previously trained deep learning models which is considered in step S12.

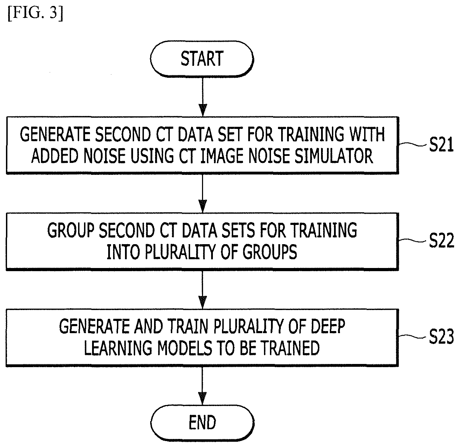

In this case, the description of the method of training the plurality of deep learning models to be trained, that is, the method for training a deep learning model may be more easily understood with reference to the following FIG. 3.

In the above-description, steps S11 to S13 may be further divided into additional steps or combined as smaller steps depending on an implementation example of the present disclosure. Further, some steps may be omitted if necessary and the order of steps may be changed.

FIG. 3 is a view illustrating an example of a schematic operation flow for a deep learning model training method for reducing a CT image noise based on deep learning by a deep learning based CT image noise reducing apparatus according to an exemplary embodiment of the present disclosure. That is, FIG. 3 is a view illustrating an example of a schematic operation flow of a method of training a deep learning model to be trained considered in the present disclosure.

The deep learning model training method for reducing a CT image noise based on deep learning illustrated in FIG. 3 may be performed by the above-described deep learning based CT image noise reducing apparatus (this apparatus, 10). Accordingly, even though description is omitted, the description for the deep learning based CT image noise reducing apparatus (this apparatus, 10) may also be applied to the description for the deep learning model training method for reducing a CT image noise based on deep learning in the same manner.

Referring to FIG. 3, in step S21, the training unit 14 may input the input CT image to the CT image noise simulator to train the deep learning models included in this apparatus 10 before the extracting unit 11 extracts test information from the input CT image in the above-described step S11 of FIG. 2.

That is, in step S21, the training unit 14 may provide the input CT image as an input of the CT image noise simulator.

Specifically, in step S21, the CT image noise simulator may generate a composite Sinogram with an original image of the first CT data set for training as an input. Further, the CT image noise simulator applies a noise model having a predetermined level (a desired level) of noise to the composite Sinogram to generate a noise component composite Sinogram. Further, the CT image noise simulator may generate a noise component CT image based on the generated noise component composite Sinogram. Further, the CT image noise simulator may generate a composite high noise CT image by adding the generated noise component CT image and the original CT image.

By doing this, in step S21, the training unit 14 may generate a second CT data set for training with various levels of noises by applying the CT image noise simulator to the first CT data set for training, before the extracting unit 11 extracts the test information from the input CT image.

That is, in step S21, the training unit 14 receives the original image using the CT image noise simulator to generate different CT data sets for training according to the plurality of noise levels.

Here, the second CT data set for training may be formed of a pair of a composite high noise CT image and a composite noise component CT image obtained based on an original image of the first CT data set for training (derived from the original image).

The training unit 14 may train the deep learning to be trained using the pair of the composite high noise CT image and the composite noise component CT image obtained by applying the CT image noise simulator to the original image of the first CT data set for training.

Next, in step S22, the training unit 14 may extract test information from the second CT data set for training and group the second CT data sets for training into a plurality of groups according to a predetermined rule.

In this case, in step S22, the training unit 14 may group the second CT data sets for training into a plurality of groups to generate CT data sets for training for every test portion and/or every scan attribute.

Next, in step S23, the training unit 14 may generate and train a plurality of deep learning models to be trained so as to correspond to the plurality of second CT data set groups for training generated in step S22. That is, in step S23, the training unit 14 may generate and train a plurality of deep learning models to be trained so as to correspond to each of the second CT data sets for training for every group which is grouped by step S22.

Specifically, in step S23, the training unit 14 may generate a deep learning model to be trained for every test portion corresponding to each CT data set for training generated for every test portion. Further, the training unit 14 may generate and train the deep learning models to be trained for every scan attribute corresponding to the CT data set for training generated for every scan attribute.

In step s23, the training unit 14 may train the plurality of deep learning models to be trained generated in step S23 using the plurality of CT data sets for training (that is, a plurality of CT data set groups for training) generated in step S22.

As a specific example, in step S23, the training unit 14 may train each deep learning model trained for every test portion and/or every scan attribute generated in step S23 so as to correspond to the CT data set for training generated for every test portion and/or the scan attribute in step S22 using the same.

Further, in step S23, in order to allow the plurality of deep learning models to be trained to have a function of extracting the noise component CT image from the input CT image input thereto, the training unit 14 may transmit a composite high noise CT image for every CT image as an input of the deep learning model to be trained, in the second CT data set for training for every group grouped in step S22 and repeatedly train the deep learning model to be trained so as to minimize a difference between the composite noise component CT image and the output of the deep learning model to be trained.

In other words, the training unit 14 may transmit a composite high noise CT image for every input CT image as an input of each of the plurality of deep learning models to be trained and repeatedly train each of the plurality of deep learning models to be trained so as to minimize a difference between the composite noise component CT image and the output of the deep learning model to be trained.

In step S23, the training unit 14 may repeatedly train the deep learning models to be trained generated for every test portion using the CT data set for training generated for every test portion. Further, the training unit 14 may repeatedly train each of the deep learning models to be trained generated for every scan attribute using the CT data set for training generated for every scan attribute.

By doing this, the plurality of deep learning models to be trained considered in this apparatus 10 may include a deep learning model to be trained generated for every test portion and a deep learning model to be trained generated for every scan attribute.

In the above-description, steps S21 to S23 may be further divided into additional steps or combined as smaller steps depending on an implementation example of the present disclosure. Further, some steps may be omitted if necessary and the order of steps may be changed.

The deep learning based CT image noise reducing method and the deep learning model training method for reducing a CT image noise based on deep learning according to the exemplary embodiment of the present disclosure may be implemented in the form of a program instruction which can be executed by various computer units to be recorded in a computer readable medium. The computer readable medium may include solely a program instruction, a data file, and a data structure or a combination thereof. The program instruction recorded in the medium may be specifically designed or constructed for the present invention or known to those skilled in the art of a computer software to be used. Examples of the computer readable recording medium include a magnetic media such as a hard disk, a floppy disk, or a magnetic tape, an optical media such as a CD-ROM or a DVD, a magneto-optical media such as a floptical disk, and a hardware device which is specifically configured to store and execute the program instruction, such as a ROM, a RAM, and a flash memory. Examples of the program instruction include not only a machine language code which is created by a compiler but also a high level language code which may be executed by a computer using an interpreter. The hardware device may operate as one or more software modules in order to perform the operation of the present invention and vice versa.

Further, the above-described deep learning based CT image noise reducing method and the deep learning model training method for reducing a CT image noise based on deep learning may be implemented in the form of a computer program or an application executed by a computer which is stored in a recording medium.

The above-description of the present disclosure is illustrative only and it is understood by those skilled in the art that the present disclosure may be easily modified to another specific type. Thus, it is to be appreciated that the embodiments described above are intended to be illustrative in every sense, and not restrictive. For example, each component which is described as a singular form may be divided to be implemented and similarly, components which are described as a divided form may be combined to be implemented.

The scope of the present invention is only limited by the appended claims.

## Claims

1. A deep learning based CT image noise reducing method, comprising:
extracting (S11) test information from an input CT image;
selecting (S12) at least one deep learning model corresponding to the test information, among a plurality of previously trained deep learning models; and
outputting (S13) a CT image with a reduced noise with respect to the input CT image with the input CT image as an input of the at least one selected deep learning model,
wherein the plurality of previously trained deep learning models is the plurality of deep learning models to be trained which is trained by performing,
**characterised by**
generating (S21) a second CT data set for training with a noise added thereto with respect to a first CT data set for training by applying a CT image noise simulator to the first CT data set for training, wherein the second CT data set for training is formed of a pair of a composite high noise CT image and a composite noise component CT image obtained based on an original image of the first CT data set for training,
grouping (S22) the second CT data set for training by extracting test information from the second CT data set for training; and
training (S23) a plurality of generated deep learning models to be trained using the grouped second CT data set for training,
wherein in the outputting, the at least one selected deep learning model extracts a noise component CT image from the input CT image with the input CT image as an input of the at least one selected deep learning model and a predetermined value is multiplied with the extracted noise component CT image to be subtracted from the input CT image to output the CT image with a reduced noise.

2. The deep learning based CT image noise reducing method according to claim 1, wherein in the generating, the CT image noise simulator performs:
generating a composite Sinogram with an original image of the first CT data set for training as an input;
generating a noise component composite Sinogram by applying a noise model with a predetermined level of noise to the composite Sinogram;
generating a noise component CT image based on the generated noise component composite Sinogram; and
generating a composite high noise CT image by adding the generated noise component CT image and the original CT image.

3. The deep learning based CT image noise reducing method according to claim 1, wherein in the training, in order to allow the plurality of deep learning models to be trained to have a function of extracting a noise component CT image from the input CT image input thereto, a composite high noise CT image for every CT image is transmitted as an input of the deep learning model to be trained, in the grouped second CT data set for training for every group and the deep learning model to be trained is repeatedly trained so as to minimize a difference between a composite noise component CT image and an output of the deep learning model to be trained.

4. A deep learning based CT image noise reducing apparatus, comprising:
an extracting unit (11) which is configured to extract test information from an input CT image;
a selecting unit (12) which is configured to select at least one deep learning model corresponding to the test information, among a plurality of previously trained deep learning models;
an output unit (13) which is configured to output an input CT image with a reduced noise with respect to the input image with the input CT image as an input of the at least one selected deep learning model; and
a training unit (14) which is configured to generate and train a plurality of deep learning models to be trained,
**characterised in that** the training unit (14) is further configured to:
generate a second CT data set for training to which a noise is added by applying a CT image noise simulator to a first CT data set for training, wherein the second CT data set for training is formed of a pair of a composite high noise CT image and a composite noise component CT image obtained based on an original image of the first CT data set for training,
extract test information from the second CT data set for training,
group the second CT data set for training into a plurality of groups according to a predetermined rule, and
generate and train a plurality of deep learning models to be trained so as to correspond to each grouped second CT data set for training for every group,
wherein the plurality of previously trained deep learning models is the plurality of deep learning models to be trained which is trained by the training unit (14),
wherein the output unit (13) is configured to allow the at least one selected deep learning model to extract a noise component CT image from the input CT image with the input CT image as an input of the at least one selected deep learning model and multiplies a predetermined value with the extracted noise component CT image to be subtracted from the input CT image to output the CT image with a reduced noise.

5. The deep learning based CT image noise reducing apparatus according to claim 4, wherein the CT image noise simulator generates a composite Sinogram with an original image of the first CT data set for training as an input, generates a noise component composite Sinogram by applying a noise model with a predetermined level of noise to the composite Sinogram, generates a noise component CT image based on the generated noise component composite Sinogram, and generates a composite high noise CT image by adding the generated noise component CT image and the original CT image.

6. The deep learning based CT image noise reducing apparatus according to claim 4, wherein in order to allow the plurality of deep learning models to be trained to have a function of extracting a noise component CT image from the input CT image input thereto, the training unit (14) is configured to transmit a composite high noise CT image for every CT image as an input of the deep learning model to be trained, in the grouped second CT data set for training for every group and repeatedly trains the deep learning model to be trained so as to minimize a difference between a composite noise component CT image and an output of the deep learning model to be trained.

7. A computer readable recording medium in which a program allowing a computer to execute the method according to any one of claim 1 to claim 3 is recorded.

## Patentansprüche

1. Verfahren für auf Tiefenlernen basierende CT-Bildrauschunterdrückung, das aufweist:
Extrahieren (S11) von Testinformationen aus einem eingegebenen CT-Bild;
Auswählen (S12) mindestens eines Tiefenlernmodells, das den Testinformationen entspricht, aus einer Mehrzahl von zuvor trainierten Tiefenlernmodellen; und
Ausgeben (S13) eines CT-Bildes mit einem unterdrückten Rauschen in Bezug auf das eingegebene CT-Bild, wobei das eingegebene CT-Bild eine Eingabe des mindestens einen ausgewählten Tiefenlernmodells ist,
wobei die Mehrzahl von zuvor trainierten Tiefenlernmodellen die Mehrzahl der zu trainierenden Tiefenlernmodellen ist, die durch Ausführung trainiert wird,
**gekennzeichnet durch**
Erzeugen (S21) eines zweiten CT-Datensatzes zum Trainieren mit einem dazu addierten Rauschen in Bezug auf einen ersten CT-Datensatz zum Trainieren durch Anwenden eines CT-Bildrauschsimulators auf den ersten CT-Datensatz zum Trainieren, wobei der zweite CT-Datensatz zum Trainieren aus einem Paar aus einem zusammengesetzten CT-Bild mit hohem Rauschen und einem zusammengesetzten Rauschkomponenten-CT-Bild gebildet ist, das auf Grundlage eines Originalbildes des ersten CT-Datensatzes zum Trainieren erlangt wird,
Gruppieren (S22) des zweiten CT-Datensatzes zum Trainieren durch Extrahieren von Testinformationen aus dem zweiten CT-Datensatz zum Trainieren; und
Trainieren (S23) einer Mehrzahl von erzeugten zu trainierenden Tiefenlernmodellen unter Verwendung des gruppierten zweiten CT-Datensatzes zum Trainieren,
wobei bei der Ausgabe das mindestens eine ausgewählte Tiefenlernmodell ein Rauschkomponenten-CT-Bild aus dem eingegebenen CT-Bild extrahiert, wobei das eingegebene CT-Bild eine Eingabe des mindestens einen ausgewählten Tiefenlernmodells ist und ein vorbestimmter Wert mit dem extrahierten Rauschkomponenten-CT-Bild zur Subtraktion von dem eingegebenen CT-Bild multipliziert wird, um das CT-Bild mit einem unterdrückten Rauschen auszugeben.

2. Verfahren für auf Tiefenlernen basierende CT-Bildrauschunterdrückung nach Anspruch 1, wobei der CT-Bildrauschsimulator bei der Erzeugung durchführt:
Erzeugen eines zusammengesetzten Sinogramms mit einem Originalbild des ersten CT-Datensatzes zum Trainieren als eine Eingabe;
Erzeugen eines zusammengesetzten Rauschkomponenten-Sinogramms durch Anwenden eines Rauschmodells mit einem vorbestimmten Rauschpegel auf das zusammengesetzte Sinogramm;
Erzeugen eines Rauschkomponenten-CT-Bildes auf Grundlage des erzeugten zusammengesetzten Rauschkomponenten-Sinogramms; und
Erzeugen eines zusammengesetzten CT-Bildes mit hohem Rauschen durch Addieren des erzeugten Rauschkomponenten-CT-Bildes und des Original-CT-Bildes.

3. Verfahren für auf Tiefenlernen basierende CT-Bildrauschunterdrückung nach Anspruch 1, wobei beim Trainieren, damit die Mehrzahl der zu trainierenden Tiefenlernmodelle eine Funktion zum Extrahieren eines Rauschkomponenten-CT-Bildes aus dem darin eingegebenen CT-Bild haben kann, ein zusammengesetztes CT-Bild mit hohem Rauschen für jedes CT-Bild als eine Eingabe des zu trainierenden Tiefenlernmodells übertragen wird in dem gruppierten zweiten CT-Datensatz zum Trainieren für jede Gruppe und das zu trainierende Tiefenlernmodell wiederholt so trainiert wird, dass eine Differenz zwischen einem zusammengesetzten Rauschkomponenten-CT-Bild und einer Ausgabe des zu trainierenden Tiefenlernmodells minimiert wird.

4. Vorrichtung für auf Tiefenlernen basierende CT-Bildrauschunterdrückung, die aufweist:
eine Extraktionseinheit (11), die dazu ausgelegt ist, Testinformationen aus einem eingegebenen CT-Bild zu extrahieren;
eine Auswahleinheit (12), die dazu ausgelegt ist, mindestens ein Tiefenlernmodell, das den Testinformationen entspricht, aus einer Mehrzahl von zuvor trainierten Tiefenlernmodellen auszuwählen;
eine Ausgabeeinheit (13), die dazu ausgelegt ist, ein CT-Bild mit einem unterdrückten Rauschen in Bezug auf das eingegebene CT-Bild auszugeben, wobei das eingegebene CT-Bild eine Eingabe des mindestens einen ausgewählten Tiefenlernmodells ist; und
eine Trainingseinheit (14), die dazu ausgelegt ist, eine Mehrzahl von zu trainierenden Tiefenlernmodellen zu erzeugen und zu trainieren,
**dadurch gekennzeichnet, dass** die Trainingseinheit (14) ferner ausgelegt ist zum:
Erzeugen eines zweiten CT-Datensatzes zum Trainieren mit einem dazu addierten Rauschen durch Anwenden eines CT-Bildrauschsimulators auf einen ersten CT-Datensatz zum Trainieren, wobei der zweite CT-Datensatz zum Trainieren aus einem Paar aus einem zusammengesetzten CT-Bild mit hohem Rauschen und einem zusammengesetzten Rauschkomponenten-CT-Bild gebildet ist, das auf Grundlage eines Originalbildes des ersten CT-Datensatzes zum Trainieren erlangt wird,
Extrahieren von Testinformationen aus dem zweiten CT-Datensatz zum Trainieren,
Gruppieren des zweiten CT-Datensatzes zum Trainieren in eine Mehrzahl von Gruppen gemäß einer vorbestimmten Regel, und
Erzeugen und Trainieren einer Mehrzahl von zu trainierenden Tiefenlernmodellen derart, dass sie jedem gruppierten zweiten CT-Datensatz zum Trainieren entsprechen, für jede Gruppe,
wobei die Mehrzahl von zuvor trainierten Tiefenlernmodellen die Mehrzahl von zu trainierenden Tiefenlernmodellen ist, die von der Trainingseinheit (14) trainiert wird,
wobei die Ausgabeeinheit (13) dazu ausgelegt ist, dem mindestens einen ausgewählten Tiefenlernmodell zu ermöglichen, ein Rauschkomponenten-CT-Bild aus dem eingegebenen CT-Bild zu extrahieren, wobei das eingegebene CT-Bild eine Eingabe des mindestens einen ausgewählten Tiefenlernmodells ist und ein vorbestimmter Wert mit dem extrahierten Rauschkomponenten-CT-Bild zur Subtraktion von dem eingegebenen CT-Bild multipliziert wird, um das CT-Bild mit einem unterdrückten Rauschen auszugeben.

5. Vorrichtung für auf Tiefenlernen basierende CT-Bildrauschunterdrückung nach Anspruch 4, wobei der CT-Bild-Rauschsimulator ein zusammengesetztes Sinogramm mit einem Originalbild des ersten CT-Datensatzes zum Trainieren als eine Eingabe erzeugt, ein zusammengesetztes Rauschkomponenten-Sinogramm durch Anwenden eines Rauschmodells mit einem vorbestimmten Rauschpegel auf das zusammengesetzte Sinogramm erzeugt, ein Rauschkomponenten-CT-Bild auf Grundlage des erzeugten zusammengesetzten Rauschkomponenten-Sinogramms erzeugt und ein zusammengesetztes CT-Bild mit hohem Rauschen durch Addieren des erzeugten Rauschkomponenten-CT-Bildes und des Original-CT-Bildes erzeugt.

6. Vorrichtung für auf Tiefenlernen basierende CT-Bildrauschunterdrückung nach Anspruch 4, wobei, damit die Mehrzahl der zu trainierenden Tiefenlernmodelle eine Funktion zum Extrahieren eines Rauschkomponenten-CT-Bildes aus dem eingegebenen CT-Bild haben kann, die Trainingseinheit (14) dazu ausgelegt ist, ein zusammengesetztes CT-Bild mit hohem Rauschen für jedes CT-Bild als eine Eingabe des zu trainierenden Tiefenlernmodells in dem gruppierten zweiten CT-Datensatz zum Trainieren für jede Gruppe zu übertragen und das zu trainierende Tiefenlernmodell wiederholt so zu trainieren, dass eine Differenz zwischen einem zusammengesetzten Rauschkomponenten-CT-Bild und einer Ausgabe des zu trainierenden Tiefenlernmodells minimiert wird.

7. Computerlesbares Aufzeichnungsmedium, auf dem ein Programm aufgezeichnet ist, das es einem Computer ermöglicht, das Verfahren nach einem der Ansprüche 1 bis 3 auszuführen.

## Revendications

1. Un procédé de réduction du bruit d'images de tomodensitométrie, CT, basé sur l'apprentissage profond, comprenant les étapes consistant à :
extraire (S11) des informations de test à partir d'une image CT d'entrée;
sélectionner (S12) au moins un modèle d'apprentissage profond correspondant aux informations de test, parmi une pluralité de modèles d'apprentissage profond précédemment entraînés ; et
produire (S13) une image CT avec un bruit réduit par rapport à l'image CT d'entrée, l'image CT d'entrée servant d'entrée audit au moins un modèle d'apprentissage profond sélectionné,
dans lequel la pluralité de modèles d'apprentissage profond précédemment entraînés est la pluralité de modèles d'apprentissage profond à entrainer qui sont entraînés par l'exécution,
**caractérisé par** la génération (S21) d'un second ensemble de données CT pour l'entrainement avec un bruit ajouté par rapport à un premier ensemble de données CT pour l'entrainement en appliquant un simulateur de bruit d'image CT au premier ensemble de données CT pour l'entrainement, dans lequel le second ensemble de données CT pour l'entrainement consiste en une paire formée d'une image composite CT à bruit élevé et d'une image composite CT à composante de bruit, obtenues à partir d'une image originale du premier ensemble de données CT pour l'entrainement,
regrouper (S22) le second ensemble de données CT pour l'entrainement en extrayant des informations de test du second ensemble de données CT pour l'entrainement ; et
entrainer (S23) une pluralité de modèles d'apprentissage profond générés à entrainer en utilisant le second ensemble de données CT pour l'entrainement,
dans lequel, lors de la génération, ledit au moins un modèle d'apprentissage profond sélectionné extrait une image CT à composante de bruit de l'image CT d'entrée, l'image CT d'entrée servant d'entrée audit au moins un modèle d'apprentissage profond sélectionné, et une valeur prédéterminée est multipliée par l'image CT à composante de bruit extraite pour être soustraite de l'image CT d'entrée afin de produire l'image CT avec un bruit réduit.

2. Le procédé de réduction du bruit d'images CT basé sur l'apprentissage profond selon la revendication 1, dans lequel, lors de la génération, le simulateur de bruit d'image CT effectue les opérations consistant à :
générer un sinogramme composite avec une image originale du premier ensemble de données CT pour l'entrainement en tant qu'entrée ;
générer un sinogramme composite à composante de bruit en appliquant un modèle de bruit avec un niveau de bruit prédéterminé au sinogramme composite ;
générer une image CT à composante de bruit sur la base du sinogramme composite à composante de bruit généré ; et
générer une image composite CT à bruit élevé en additionnant l'image CT à bruit générée et l'image CT originale.

3. Le procédé de réduction du bruit d'images CT basé sur l'apprentissage profond selon la revendication 1, dans lequel, lors de l'entrainement, afin de permettre à la pluralité de modèles d'apprentissage profond d'être entraînés pour avoir une fonction d'extraction d'une image CT à composante de bruit à partir de l'image CT d'entrée entrée dans ceux-ci, une image composite CT à bruit élevé pour chaque image CT est transmise en tant qu'entrée du modèle d'apprentissage profond à entrainer, dans le second ensemble de données CT groupées pour l'entrainement de chaque groupe, et le modèle d'apprentissage profond à entrainer est entrainé de manière répétée afin de minimiser une différence entre une image composite CT à composante de bruit et une sortie du modèle d'apprentissage profond à entrainer.

4. Un appareil de réduction du bruit des images CT basé sur l'apprentissage profond, comprenant :
une unité d'extraction (11) configurée pour extraire des informations de test à partir d'une image CT d'entrée ;
une unité de sélection (12) configurée pour sélectionner au moins un modèle d'apprentissage profond correspondant aux informations de test, parmi une pluralité de modèles d'apprentissage profond précédemment entrainés ;
une unité de sortie (13) configurée pour produire une image CT d'entrée avec un bruit réduit par rapport à l'image d'entrée, l'image CT d'entrée servant d'entrée audit au moins un modèle d'apprentissage profond sélectionné ; et
une unité d'entrainement (14) qui est configurée pour générer et entrainer une pluralité de modèles d'apprentissage profond à entrainer,
**caractérisé en ce que** l'unité d'entrainement(14) est en outre configurée pour :
générer un second ensemble de données CT pour l'entrainement auquel un bruit est ajouté en appliquant un simulateur de bruit d'image CT à un premier ensemble de données CT pour l'entrainement, dans lequel le second ensemble de données CT pour l'entrainemnet consiste en une paire formée d'une image composite CT à bruit élevé et d'une image composite CT à composante de bruit, obtenues à partir d'une image originale du premier ensemble de données CT pour l'entrainement,
extraire des informations de test du second ensemble de données CT pour l'entrainement,
regrouper le second ensemble de données CT pour l'entrainement en plusieurs groupes selon une règle prédéterminée, et
générer et former une pluralité de modèles d'apprentissage profond à entrainer de manière à correspondre à chaque second ensemble de données CT groupé pour l'entrainement pour chaque groupe,
dans lequel la pluralité de modèles d'apprentissage profond précédemment entraînés est la pluralité de modèles d'apprentissage profond à entrainer qui sont entraînés par l'unité d'entrainement (14),
dans lequel l'unité de sortie (13) est configurée pour permettre audit au moins un modèle d'apprentissage profond sélectionné d'extraire une image CT à composante de bruit de l'image CT d'entrée, l'image CT d'entrée servant d'entrée audit au moins un modèle d'apprentissage profond sélectionné, et de multiplier une valeur prédéterminée avec l'image CT à composante de bruit extraite à soustraire de l'image CT d'entrée afin de produire l'image CT avec un bruit réduit.

5. L'appareil de réduction du bruit des images CT basé sur l'apprentissage profond selon la revendication 4, dans lequel le simulateur de bruit d'image CT génère un sinogramme composite avec une image originale du premier ensemble de données CT pour l'entrainement en tant qu'entrée, génère un sinogramme composite à composante de bruit en appliquant un modèle de bruit avec un niveau de bruit prédéterminé au sinogramme composite, génère une image CTà composante de bruit sur la base du sinogramme composite à composante de bruit généré, et génère une image composite CT à bruit élevé en additionant l'image CT à bruit générée et l'image CT originale.

6. L'appareil de réduction du bruit des images CT basé sur l'apprentissage profond selon la revendication 4, dans lequel, afin de permettre à la pluralité de modèles d'apprentissage profond d'être entraînés pour avoir une fonction d'extraction d'une image CT à composante de bruit à partir de l'image CT d'entrée entrée dans ceux-ci, l'unité d'entrainement (14) est configurée pour transmettre une image composite CT à bruit élevé pour chaque image CT en tant qu'entrée du modèle d'apprentissage profond à entrainer, dans le second ensemble de données CT groupées pour l'entrainement de chaque groupe et entrainer de manière répétée le modèle d'apprentissage profond à entrainer afin de minimiser une différence entre une image composite CT à composante de bruit et une sortie du modèle d'apprentissage profond à entrainer.

7. Un support d'enregistrement lisible par ordinateur stockant un programme permettant à un ordinateur de mettre en oeuvre le procédé selon l'une quelconque des revendications 1 à 3.
